# FASCICULE DE BREVET EUROPEEN

(11) **EP 0 385 929 B1**
(45) Date de publication et mention de la délivrance du brevet: **10.08.1994**
(21) Numéro de dépôt: 90810092.8
(22) Date de dépôt: 09.02.1990
(51) Int. Cl.: A61B 17/60

(54) **Fixateur externe avec amortissement contrôlable**
Vorrichtung zum externen Festlegen von Knochenfragmenten mit kontrollierbarer Dämpfung
External fixator with controllable damping

(30) Priorité: 27.02.1989 CH 698/89
(43) Date de publication de la demande: 05.09.1990
(73) Titulaire: JAQUET ORTHOPEDIE S.A., CH-1228 Plan-les-Ouates (CH)
(72) Inventeur: Wagenknecht, Marcel, CH-1219 Le Lignon (CH); Lazo de Zbikowski Juan (Dr.), E-41011 Séville (ES); Canadell José (Dr.), E-31002 Pampelune (ES)
(74) Mandataire: Dietlin, Henri

(56) Documents cités:
- EP-A- 0 256 984
- WO-A-87/07134
- WO-A-88/02618
- FR-A- 2 322 579
- GB-A- 2 104 782

## Description

La présente invention est du domaine de la traumatologie et de l'orthopédie et a plus précisément pour objet un appareil externe de fixation osseuse au moyen de fiches introduites dans l'os.

Par traumatologie, on entend la chirurgie des accidents et par orthopédie la correction de malformations, congénitales ou subséquentes à une consolidation osseuse selon un positionnement incorrect.

Depuis de nombreuses années on a développé plusieurs dispositifs autorisant l'allongement ou la compression d'os, qu'il y ait fracture ou corticotomie ou non.

L'invention se rapporte à des fixateurs externes, reliés à des groupes de fiches insérées dans l'os et autorisant un déplacement longitudinal par rapport à l'os des groupes de fiches, tout en maintenant en place les positions angulaires.

Dans l'exposé, on parle d'os fracturé, mais il va sans dire qu'il peut s'agir aussi d'un os volontairement sectionné, afin de l'allonger par exemple en orthopédie. Il peut s'agir également d'un os non fracturé ou sectionné, dont on désire varier la longueur, par compression ou allongement, comme cela est pratiqué chez les sujets jeunes.

Selon une technique connue depuis plusieures années, les fiches sont reliées à une barre de fixation externe, disposée sensiblement parallèlement à l'os fracturé. Chaque groupe de fiches insérées dans un fragment d'os est serré dans un support orientable par rapport à la barre de fixation, et solidaire d'une bride de fixation sur celle-ci. Pour obtenir la compression ou l'allongement désiré au cours de la consolidation osseuse, on peut soit déplacer l'une des brides le long de la barre, soit utiliser une barre télescopique.

On sait également qu'au cours de la consolidation osseuse, il est avantageux d'autoriser un léger mouvement de va-et-vient dit de biocompression, qui permet aux forces naturelles des muscles et des charges supportées d'agir sur la fracture, de manière à stimuler la croissance de l'os.

Dans le brevet américain US-A-4 502 473 par exemple est décrit un fixateur externe comportant deux groupes de fiches insérées dans l'os et solidaires de brides de fixation, l'une des brides de fixation des fiches étant apte à se déplacer le long d'une barre de fixation disposée parallèlement à l'os. Un élément élastique est prévu dans la pièce de raccordement des deux brides et assure une compression de la fracture sur une course limitée. En outre on peut équiper ce fixateur de moyens pneumatiques ou mécaniques permettant d'assurer une stimulation passive.

Par le brevet français FR-A-2 517 195 (US-A-4 488 542) on connaît un dispositif pour la correction et la réduction de fragments osseux comportant une barre rigide ajustable en longeur sur laquelle sont assujettis deux supports de fiches insérées dans les fragments osseux. Dans une variante le déplacement longitudinal est obtenu par un mouvement télescopique entre deux tubes polygonaux, l'un étant solidaire d'une vis sans fin et l'autre d'un écrou. Un élément de guidage coulissant est intercalé entre ces deux éléments télescopiques pour empêcher tout jeu et pour permettre un glissement sans friction de l'un des éléments par rapport à l'autre.

La présente invention vise à permettre le contrôle de la force appliquée lors du déplacement relatif des deux groupes de fiches insérés dans les fragments osseux, qu'il s'agisse de compression ou d'allongement, afin d'éviter d'endommager le cal osseux en formation lorsque la force de déplacement est appliquée.

L'invention a pour objet un fixateur externe pour la correction et la réduction de fragments osseux comportant une barre rigide disposée sensiblement parallèlement à l'os, au moins deux supports d'orientation des fiches insérées dans les fragments osseux, l'écartement entre lesdits supports étant ajustable au moyen d'un organe de déplacement évitant toute rotation entre les supports, la barre comportant un élément d'amortissement du déplacement longitudinal.

Elle est caractérisée par le fait que :
a) le premier support est solidaire d'un carter disposé en bout de la barre rigide et apte à se déplacer par rapport à un coulisseau fixé à une extrémité de la barre, de manière à obtenir un mouvement de va-et-vient longitudinal du carter par rapport à la barre et éviter tout déplacement angulaire;
b) le second support est déplaçable longitudinalement par rapport au premier support au moyen dudit organe de déplacement; et
c) l'élément d'amortissement coopère avec des moyens de réglage de la force d'amortissement disposés extérieurement en bout du fixateur.

Grâce à la combinaison des éléments constructifs mentionnés, il est possible d'obtenir une barre de fixation permettant de déplacer un fragment osseux tout au cours de la consolidation osseuse, sans risque de déchirure du cal en formation, puisque le chirurgien peut connaître la force appliquée au moment du déplacement, et de plus en autorisant un mouvement de biocompression favorisant la formation du cal osseux et choisi en fonction de facteurs personnels à chaque patient.

Dans une forme d'exécution préférentielle, le coulisseau présente des formes destinées à coopérer avec des découpures correspondantes dans une pièce de liaison, de manière à éviter tout déplacement angulaire. Ce coulisseau comporte une extrémité de section polygonale, destinée à coulisser par rapport au carter dans un roulement à aiguilles. On prévoit avantageusement de limiter de manière prédéterminée l'amplitude du va-et-vient longitudinal autorisée.

Les supports d'orientation des fiches sont constitués par des mâchoires disposées de part et d'autre de la barre coopérant avec un organe arqué de déflection apte à autoriser le pivotement angulaire d'étaux de serrage des fiches osseuses. On peut, selon les besoins, prévoir un renvoi angulaire inséré entre l'organe arqué de déflection et l'étau.

Dans une forme d'exécution préférentielle, l'élément d'amortissement est constitué par un élément élastique comprimé entre deux portées, par exemple un ressort boudin, et des moyens sont prévus pour varier sa compression. Pour permettre au praticien de connaître la force appliquée, une tige pouvant faire saillie à l'extérieur de la barre comporte des repères gradués, aptes à indiquer des valeurs de forces.

De manière connue, on utilisera une barre tubulaire comportant extérieurement des graduations permettant de mesurer le déplacement effectué au moyen de l'organe de déplacement. Dans une variante préférentielle, la barre est de forme générale quadrangulaire avec des pans coupés à 20° de manière à assurer le positionnement des supports d'orientation des fiches.

Selon une première forme d'exécution, l'organe de déplacement apte à assurer le déplacement de l'un des supports est constitué par un ensemble de déplacement extérieur à la barre et comportant des moyens de fixation à la barre et des moyens de déplacement longitudinal du dit support.

Dans une autre forme d'exécution proposée, l'organe de déplacement apte à assurer le déplacement de l'un des supports est constitué par un dispositif intérieur à une barre formée de tubes télescopiques. Ce dispositif peut comprendre un engrenage cônique dont l'un des arbres est apte à être entraîné depuis l'extérieur et dont l'autre arbre est solidaire d'une tige filetée apte à coopérer avec le tube télescopique à déplacer.

Lorsque le patient doit rester alité, on peut utiliser le fixateur externe décrit en y adjoignant un ensemble moteur d'entraînement, à l'encontre de l'élément d'amortissement, permettant de simuler les mouvements de biocompression.

Pour que la gêne suscitée chez le patient par le fixateur externe soit minimum, on cherche autant que possible à diminuer l'encombrement et le poids de ses composants.

Le dessin annexé représente, à titres d'exemples non limitatifs, quelques formes d'exécution de l'objet de la présente invention.

La figure 1 est une vue schématique en perspective d'une première forme d'exécution de fixateur externe comprenant en outre un dispositif de déplacement longitudinal de l'une des brides de fixation des fiches insérées dans un fragment d'os le long d'une barre de fixation.

La figure 2 est une coupe longitudinale de la barre de fixation et du système de biocompression, représentés à la figure 1, selon les lignes II-II indiquées aux figures 4 et 5.

La figure 3 est une vue en bout de l'extrémité libre de la barre de fixation de la figure 2.

La figure 4 est une coupe transversale de l'extrémité de liaison de la barre de fixation, selon IV-IV à la figure 2.

La figure 5 est une coupe transversale du système de biocompression, selon V-V à la figure 2.

La figure 6 représente, en coupe longitudinale, une variante d'exécution de barre de fixation à éléments télescopiques.

La figure 7 est une coupe transversale selon VII-VII de la barre de fixation de la figure 6.

La figure 8 est une coupe transversale selon VIII-VIII de la barre de fixation de la figure 6.

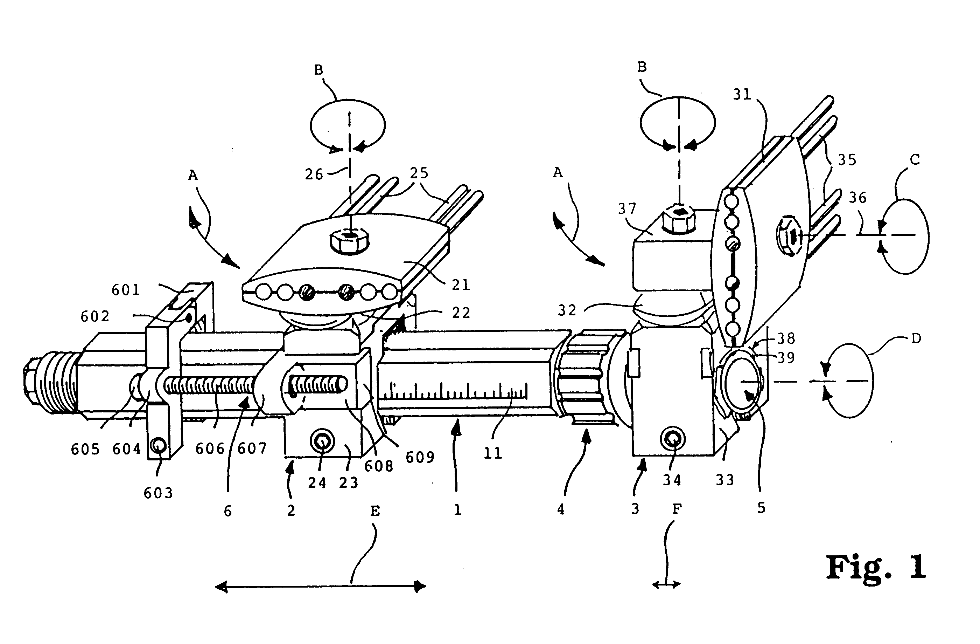

Dans la vue générale de la figure 1 on a représenté une barre de fixation externe 1, de section polygonale, sur laquelle sont placées deux brides 2 et 3 solidaires indirectement d'étaux 21 et 31 de serrage de fiches 25 et 35.

Chaque bride 2 ou 3 comporte un organe arqué de déflection 22, 32 coopérant avec une paire de mâchoires 23, 33 disposées de part et d'autre de la barre 1 sur laquelle elles sont fixées au moyen d'un dispositif de positionnement 24, 34.

L'organe arqué de déflection 22 autorise le pivotement angulaire de l'étau 21 selon la flèche A d'une part, et l'orientation continue de 0 à 360° de l'ensemble des fiches 25 par rapport à l'axe 26 de serrage, selon la flèche B d'autre part.

La bride 3 comporte en outre un organe de renvoi angulaire 37, permettant de basculer de 90° l'étau 31 et par conséquent les fiches 35. Un tel renvoi angulaire est utilisé par exemple lorsque les fiches 35 sont destinées à être insérées dans l'épiphyse d'un os (par exemple, plateau tibial ou fémoral) dont le corps diaphysaire recevrait les fiches 25. En plus du pivotement circulaire selon la flèche A et de l'orientation angulaire selon la flèche B, la bride 3 permet aussi une orientation angulaire par rapport à l'axe 36 de serrage des fiches 35, selon la flèche C.

La flèche E représente le déplacement de la bride le long de la barre, assurant l'allongement ou la compression de l'os parallèlement à la barre, tandis que la flèche F représente le mouvement limité de biocompression, réglable au moyen de l'écrou moleté 4, déplaçable entre l'extrémité de la barre 1 et le système de biocompression 5, comme on le verra en détail plus loin.

Il est à noter que le système de biocompression 5 est de forme générale cylindrique, ce qui autorise encore le positionnement angulaire de la bride 3 selon la flèche D, grâce à une pièce intermédiaire de raccord 38, dont les formes extérieures correspondent aux découpes intérieures des mâchoires 33. Le raccord 38 présente en outre une collerette vers l'extérieur 39, de part et d'autre de la bride 3, destinée à maintenir le raccord 38 dans la bride.

Dans la vue schématique de la figure 1 on a également représenté un organe 6 de déplacement de la bride 2 le long de la barre 1 qui peut comporter des repères linéaires 11, généralement gradués en millimètres.

L'organe de déplacement 6 est constitué d'une bride de fixation 601, en deux parties articulées sur un axe 602, qui est serrée sur la barre 1 par une vis 603. Il comporte en outre un support 604, solidaire en rotation de la bride 601 et présentant une ouverture de retenue de la tête 605 d'une vis-mère 606 le long de laquelle se déplace un bossage taraudé 607 venu de fabrication avec un étrier 608 muni de deux ailes 609 destinées à enserrer la partie latérale de la mâchoire 23 de la bride 2.

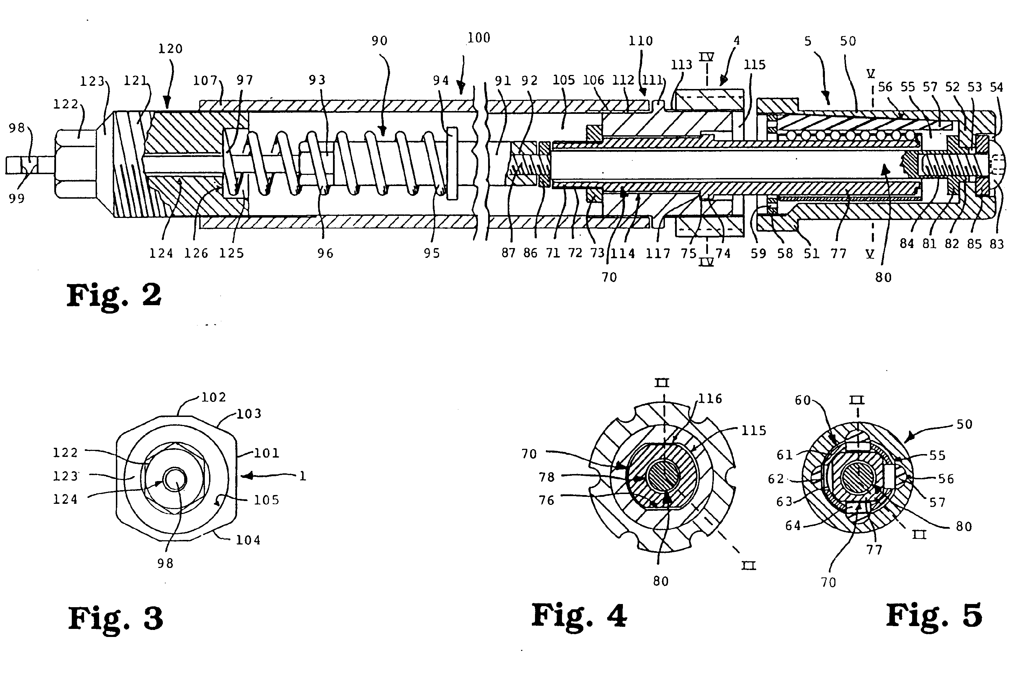

Dans la coupe longitudinale de la figure 2, on retrouve la barre de fixation externe 1 sous référence 100. Elle est, comme on l'a déjà mentionné, de section polygonale, visible dans la vue en bout de la figure 3, où l'on notera que la barre 100 comporte des faces parallèles 101 et 102, chacune suivies par des faces 103 et 104, inclinées de 20° de manière à assurer un serrage optimum de la barre dans des découpes internes de formes correspondantes des mâchoires de la bride. En variante, on peut utiliser une barre de fixation de section circulaire sur laquelle sont serrées des brides de forme correspondante.

La barre de fixation 100 est tubulaire et présente une ouverture centrale circulaire 105, terminée à chaque extrémité de la barre par un taraudage 106 et 107 respectivement.

Le taraudage 106 reçoit une pièce de liaison 110, de forme générale cylindrique, présentant une collerette médiane 111 servant de butée d'une part au filetage 112 assurant la liaison avec la barre de fixation, et plus précisément son taraudage 106, et d'autre part au filetage 113 destiné à recevoir l'écrou moleté 4 déjà mentionné.

La pièce de liaison 110 comporte une ouverture centrale circulaire 114, se terminant extérieurement dans un dégagement 115 présentant au moins une face plane 116 (mieux visible à la figure 4) destinée à empêcher toute rotation de l'arbre traversant l'ouverture 114. Le dégagement 115 crée un rebord de butée 117 dudit arbre, qui sera détaillé par la suite.

Le taraudage 107 reçoit un embout de réglage 120 de forme générale cylindrique présentant extérieurement un filetage 121 correspondant au taraudage 107. L'extrémité libre de l'embout 120 comporte des moyens de préhension, par exemple constitués par un six pans 122, venu de fabrication avec l'embout 120 et faisant suite à un rétrécissement tronconique 123.

L'embout de réglage 120 comporte une ouverture centrale circulaire 124, dans lequel peut coulisser une tige, dont l'utilité sera mentionnée plus loin. L'ouverture centrale 124 débouche dans un dégagement 125 créant un rebord de butée 126.

Le système de biocompression 5 déjà mentionné en regard de la figure 1 est détaillé en coupes longitudinale et transversale aux figures 2 et 5 respectivement.

Il est constitué d'un carter 50 de forme générale cylindrique dont une extrémité présente une collerette extérieure 51 munie d'un taraudage, tandis que l'autre extrémité est fermée par une paroi 52 munie d'un passage central 53 et d'un dégagement extérieur 54. Le carter 50 comporte une ouverture centrale 55 débouchant dans quatre fraisages inclinés 56 destinés à recevoir quatre lardons 57 présentant chacun une courbure correspondant aux fraisages 56 et une face plane inclinée longitudinalement. Les fraisages 56 sont pratiqués de manière à ce que les faces planes des lardons 57 constituent les faces de glissement d'un roulement à aiguilles 60 comportant une cage 61, de forme générale cylindrique, présentant quatre faces planes extérieures 62 le long desquels sont pratiqués des fraisages 63 en échelle, destinés à recevoir les aiguilles 64 constituant le roulement à aiguilles 60 (voir figure 5).

Les lardons 57 et la cage à aiguilles 60 sont maintenus dans l'ouverture centrale 55 du carter 50 au moyen d'une rondelle 58 présentant un filetage externe destiné à coopérer avec le taraudage intérieur pratiqué dans la collerette 51. La rondelle 58 est munie de dégagements 59 destinés à recevoir un outil de serrage de forme adéquate.

A l'intérieur de la cage à aiguilles 60 est disposé un coulisseau 70 constitué par un arbre tubulaire présentant successivement :
- une extrémité de section circulaire 71 comportant un filetage 72 destiné à recevoir un écrou de retenue 73,
- une collerette médiane 74 créant un rebord de butée 75 destiné à coopérer avec le rebord de butée 117 de la pièce de liaison 110, et dont le contour extérieur comporte des plats 76 destinés à s'engager contre les faces planes 116 de la pièce de liaison 110, et
- une extrémité de section quadrangulaire 77, destinée à coulisser dans la cage à aiguilles 60.

L'arbre tubulaire 70 présente un passage central 78 de section circulaire, destiné au passage d'un arbre central 80.

L'arbre central 80 présente à une extrémité une collerette 81 destinée à s'appuyer à l'intérieur de la paroi 52 et son extrémité 82 vient s'engager dans l'ouverture 53 du carter 50, dans lequel elle est fixée au moyen d'une vis 83 venant en prise dans une ouverture centrale taraudée 84 de l'arbre central 80. Pour assurer la fixation de l'arbre central 80 dans le carter 50, on disposera avantageusement une rondelle 85 au fond du dégagement extérieur 54 qui permet en outre de noyer partiellement la tête de la vis 83.

L'extrémité de l'arbre central 80 opposée au carter 50 du système de biocompression comporte des moyens de retenue de l'ensemble, constitués ici par un écrou 86 serré sur une partie filetée 87 terminale de l'arbre central 80. L'écrou 86 est dimensionné pour buter contre l'extrémité de section circulaire 71 de l'arbre tubulaire 70. La partie filetée 87 permet de réaliser la liaison entre l'arbre central 80 et un ensemble 90 d'amortissement de valeur réglable.

Cet ensemble d'amortissement 90 est constitué d'une tige 91 comportant à une extrémité une ouverture centrale taraudée 92 destinée à coopérer avec la partie filetée 87 de l'arbre central. Pour réaliser cette liaison, l'ensemble d'amortissement 90 comporte des moyens de serrage constitués par exemple par une partie à six pans 93. Elle comporte encore une collerette médiane 94 contre laquelle s'appuye l'extrémité 95 d'un ressort boudin 96 dont l'autre extrémité 97 vient se loger dans le dégagement 125 et s'appuyer contre le rebord de butée 126.

L'ensemble 90 est terminé par une tige 98 destinée à coulisser dans l'ouverture centrale circulaire 124 de l'embout de réglage 120. L'extrémité de la tige 98 comporte des marques 99 dont l'utilité sera mentionnée plus loin.

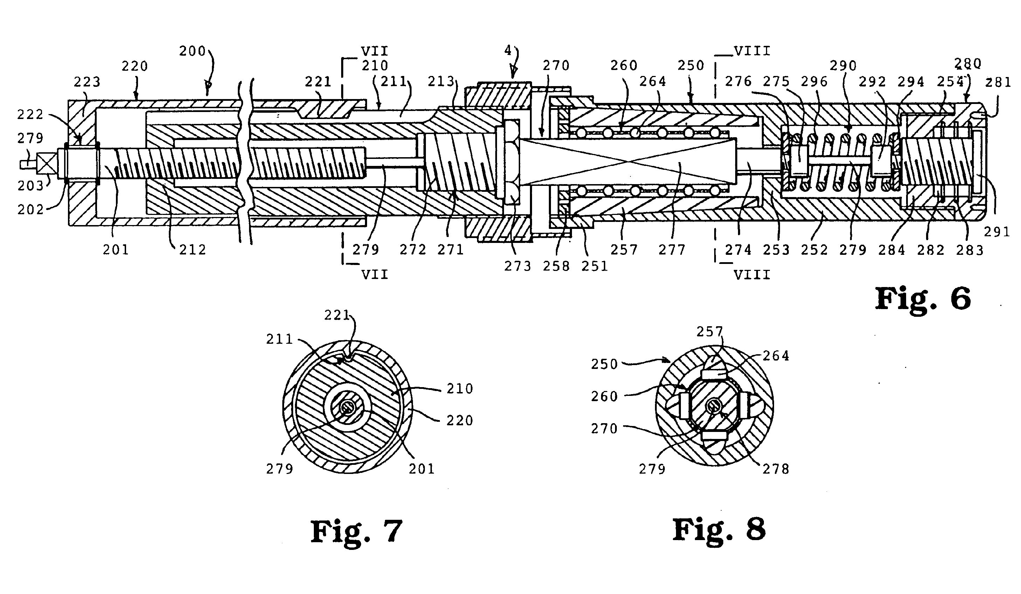

Dans la forme d'exécution représentée à la figure 6, la barre de fixation est télescopique, afin de permettre le déplacement des brides de fixation des fiches l'une par rapport à l'autre. En outre le système d'amortissement est disposé en bout du carter.

On retrouve dans cette variante le système de biocompression constitué par un carter 250 recevant dans quatre dégagements inclinés 256 des lardons 257 coopérant avec les aiguilles 264 du roulement 260 destiné à faciliter le mouvement de va-et-vient du coulisseau 270. Le roulement à aiguilles 260 est disposé entre la rondelle 258 vissée dans la collerette extérieure 251 et une paroi médiane 253 et le système d'amortissement est situé dans le prolongement 252 du carter, entre cette paroi médiane 253 et un bouchon 280 fixé dans un taraudage 254 pratiqué à l'extrémité 252 du carter.

Le coulisseau 270 présente :
- une extrémité de section circulaire 271 comportant un filetage 272 destiné à coopérer avec l'extrémité du tube intérieur 210 de l'ensemble télescopique 200, contre lequel il peut être serré grâce à la collerette à 6 pans 273 (il est à noter que cette liaison peut être effectuée par tous moyens équivalents tels que collage ou soudure);
- une extrémité de section circulaire 274 apte à coulisser dans une ouverture centrale de la paroi 253 et munie d'un taraudage destiné à recevoir une vis 275 maintenant un rondelle 276;
- une partie médiane de section quadrangulaire 277, destinée à coulisser dans la cage à aiguilles 260.

Sur toute sa longueur le coulisseau 270 peut présenter un passage central 278 (figure 8) pour le passage d'une tige 279 dont l'utilité sera mentionnée plus loin.

Le bouchon 280 est muni de dégagements externes 281 destinés à coopérer avec un outil de serrage, pour sa fixation dans le taraudage 254 pratiqué à l'extrémité du tube 253. Il comporte un évidement central 282 présentant des rainures circulaires 283 permettant de contrôler la position du système d'amortissement 290 (et par conséquent de mesurer la force appliquée), ainsi qu'un fond taraudé 284 recevant l'embout 291 de réglage de la force appliquée.

Le système d'amortissement 290 est constitué par un embout fileté 291, dont la partie extérieure au tube comporte des moyens de préhension non représentés au dessin. La face interne de l'embout 291 comporte un taraudage destiné à recevoir une vis 292 maintenant une rondelle 294. Un ressort 296 est disposé entre les rondelles 276 et 294 et sa force peut être réglée par le positionnement de l'embout 291 dans le bouchon 280.

Dans la variante des figures 6 et 7, la barre de fixation 200 est constituée par un tube intérieur 210 et un tube extérieur 220, de section circulaire. Comme déjà mentionné, une échelle graduée permet de connaître le déplacement relatif des tubes. Les tubes 210 et 220 sont généralement en aluminium. En variante on peut prévoir de superposer un tube en acier inoxydable à paroi faible et une couche en fibres de carbone, afin de diminuer le poids de l'ensemble.

Pour éviter tout déplacement angulaire des tubes, le tube intérieur présente une cannelure 211 dans laquelle coulisse un ergot 221 du tube extérieur. Le mouvement relatif des deux tubes est obtenu par une tige filetée 201 maintenue dans un passage central 222 du fond 223 du tube extérieure par deux clips 202 disposés dans des rainures circulaires de part et d'autre du fond 223. La tige 279 déjà mentionnée traverse également la tige 201 et fait saillie à l'extrémité libre de celle-ci. Extérieurement à la barre, la tige 201 présente des moyens de préhensions constitués par un carré 203, ou tout autre moyen équivalent. La partie filetée de la barre 201 coopère avec un taraudage pratiqué dans la paroi latérale 212.

En variante, on peut prévoir de tourner la tige filetée 201 non en bout de l'ensemble, mais avec un dispositif situé latéralement le long de la barre et comportant un engrenage cônique ayant une roue menante solidaire de moyens de préhension et une roue menée solidaire de la tige filetée 201.

Le tube intérieur 210 comporte, à son extrémité recevant le système de biocompression, un filetage 213 destiné à coopérer avec l'écrou moleté 4, mentionné précédemment.

Il est à noter que pour la simplification du dessin, les brides et tous les éléments extérieurs à la barre de fixation ne sont pas représentés aux figures 2 et suivantes.

Pour le bien-être du patient, les différentes pièces extérieures constituant le fixateur selon l'invention ne présentent pas d'arêtes vives et sont dans leur majorité réalisées en alliage léger afin de limiter le poids de l'ensemble.

Avant d'être utilisé au cours de l'opération, le fixateur selon l'invention est assemblé de la manière représentée au dessin. Le carter de biocompression 50 est assemblé en introduisant les lardons 57 dans les fraisages inclinés 56 correspondants, puis la cage à aiguille 60 entourant l'extrémité de section quadrangulaire 77 du coulisseau 70, au centre duquel est disposé l'arbre central 80. La collerette 81 disposée à l'extrémité de cet arbre est pressée contre la paroi 52 par l'intermédiaire de la vis 83 et de la rondelle 85, de manière à maintenir l'arbre central 80.

Le système de biocompression ainsi réalisé est monté en bout d'une barre de fixation externe et son mouvement est favorisé par le système de roulement dans la cage à aiguilles 60.

Dans la forme d'exécution détaillée aux figures 2 à 5, l'écrou de retenue 73 est serré sur le filetage 72, de manière à solidariser le système de biocompression avec la barre de fixation externe, plus précisément avec la pièce de liaison 110. Les plats correspondants 116 et 76 de la pièce de liaison 110 et de la collerette médiane 74 empêchant toute rotation entre la barre de fixation et le carter de biocompression. L'écrou de butée 86 est disposé sur la partie filetée 87 terminale de l'arbre central 80, de manière à empêcher le retrait vers l'extérieur du système de biocompression.

L'ouverture centrale taraudée 92 à l'extrémité de la tige 91 est vissée sur la partie filetée 87, au moyen d'un outil correspondant à la partie six pans 93. Le ressort boudin 96 est mis en place sur la tige 91, entre la collerette médiane 94 et le rebord de butée 126 de l'embout de réglage 120.

Au cours de l'opération proprement dite, le médecin insère des groupes de fiches dans chaque fragment d'os, selon les techniques connues. Chaque groupe de fiches 25 ou 35 est maintenu dans un plan au moyen de l'étau 21 ou 31 et est positionné par rapport à la barre 1 en profitant des possibilités d'orientation selon les flèches A, B, C et D (figure 1) qui permettent de disposer la barre parallèlement à l'os à comprimer ou allonger. Il va sans dire que le dispositif de renvoi angulaire 37 est supprimé selon les cas.

La bride 2 est disposée sur la barre 1 au moyen du dispositif de positionnement 24 de manière à pouvoir être déplacée latéralement par l'organe de déplacement 6, dont la bride de fixation 601 est serrée sur la barre 1 grâce à la vis 603.

La bride 3 est rendue solidaire du système de biocompression 5, par serrage des mâchoires 33 au moyen du dispositif de positionnement 34. On remarquera que par construction, on peut facilement modifier l'angle relatif entre les deux brides, puisque la bride 3 est montée sur un élément circulaire, le carter 50 de biocompression, afin d'autoriser un réglage selon la flèche D à la figure 1.

Tout au long de la consolidation osseuse, le médecin pourra régler l'allongement ou la compression des fragments d'os en agissant sur la vis-mère 606 de déplacement latéral de la bride 2 selon la flèche E.

Il pourra encore régler :
1) la course de biocompression selon la flèche F en agissant sur l'écrou moleté 4 qui est représenté à la figure 2 en position intermédiaire et qui peut soit :
   - entrer en contact avec la face correspondante du carter 50, empêchant tout mouvement de biocompression,
   - venir s'appuyer contre la collerette médiane 111, autorisant un mouvement de biocompression, de l'ordre de 5 mm;
2) la force de la biocompression en agissant sur l'embout 120 qui permet de comprimer plus ou moins le ressort 96, les marques 99 de la tige 98 coulissant dans l'ouverture centrale 124 de l'embout autorisant la mesure de cette force. A cet effet, les marques correspondent à des valeurs exprimées en kg-force.

Un déplacement de la bride 2 de 1 mm, mesurable sur les repères linéaires 11, correspond à un déplacement de 1 mm du fragment osseux lorsqu'il y a équilibre entre la force du ressort 96 et la résistance du membre du patient à l'allongement ou à la compression. En cas d'allongement, il est à noter que l'ensemble d'amortissement 90, par son ressort 96, permet d'empêcher la déchirure du cal en formation (anticollapse) au moment où la bride 2 est déplacée. Habituellement, on réalise un déplacement de l'ordre de 1 mm par jour, mais cette valeur peut varier selon l'âge du patient et d'autres critères individuels tels que vitesse de formation du cal.

Il va sans dire que la vis-mère 606 peut être entraînée en rotation par des moyens automatiques, tels que moteur d'entraînement, sans sortir du cadre de la présente invention.

Quant à la seconde forme d'exécution représentée aux figures 6 à 8, son montage est partiellement semblable à celui décrit auparavant, sauf que le système d'amortissement 290 est introduit dans le carter 250.

Pour rendre le système de biocompression solidaire de la barre de fixation externe, on serre par la collerette 273 le carter 250 en bout du tube intérieur 210. Le réglage de la force de biocompression est effectué en actionnant l'embout 291 et est mesurable grâce aux marques 283 de l'évidement central 282 visibles au-delà de l'embout 291.

Les deux tubes télescopiques 210 et 220 sont ensuite réunis par la tige filetée 201. Comme déjà mentionné on peut créer, mécaniquement ou électriquement, les mouvements de biocompression dans le cas où le patient reste couché. A titre d'exemple, l'ensemble moteur peut être constitué d'un moteur électrique entraînant en rotation un excentrique solidaire de l'extrémité libre de la tige 279 qu'il pousse à l'encontre du ressort 296 dans un mouvement de va-et-vient.

La barre de fixation télescopique des figures 6 à 8 est mise en place au cours de l'opération, comme indiqué précédemment, et les brides de fixation sont serrées autour du carter 250 de biocompression d'une part et autour du tube extérieur 220 du tube télescopique 200.

Au cours de la consolidation osseuse, le médecin pourra régler l'allongement ou la compression des fragments d'os en agissant sur la tige filetée 201 qui assure ainsi le mouvement relatif entre les tubes télescopiques 210 et 220.

Il est à noter que les déplacements de biocompression sont indépendants de l'allongement ou de la compression. Comme précédemment décrit, l'amplitude des mouvements de biocompression est réglable au moyen de l'écrou molleté 4.

Dès que l'on applique une force pour éviter la déchirure du cal en formation, tout mouvement de l'un des tubes télescopiques par rapport à l'autre se traduit sur l'os par un déplacement différent de celui indiqué sur l'échelle graduée du tube intérieur 210. En effet, il faut d'abord équilibrer la compression du ressort avec les réactions de résistance des muscles et des tissus, et seulement ensuite un déplacement relatif des tubes télescopiques de 1 mm se traduit par un déplacement équivalent des groupes de fiches insérées dans l'os.

Il va sans dire que le fixateur selon l'invention peut être utilisé pour déplacer un segment d'os entre deux parties d'un os long. Dans ce cas le fixateur comprend deux supports fixes et un support déplaçable, solidaire du fragment d'os. On peut également prévoir d'utiliser certains des éléments décrits dans un fixateur destiné à maintenir des fiches osseuses en place, sans compression ni élongation au niveau de la fracture.

Il est bien sûr possible, sans sortir du cadre de la présente invention, d'utiliser la barre de fixation décrite précédemment avec des supports de fiches à rotule, permettant le positionnement des fiches dans le plan désiré, solidaires d'une part de la barre de fixation et d'autre part du carter de biocompression.

## Revendications

1. Fixateur externe pour la correction et la réduction de fragments osseux comportant une barre rigide (100,200) disposée sensiblement parallèlement à l'os, au moins deux supports (2,3) d'orientation des fiches (25,35) insérées dans les fragments osseux, l'écartement entre lesdits supports étant ajustable au moyen d'un organe de déplacement (6,201) évitant toute rotation entre les supports, la barre comportant un élément d'amortissement (90,290) du déplacement longitudinal, caractérisé par le fait que :
a) le premier support (3) est solidaire d'un carter (50, 250) disposé en bout de la barre rigide (100,200) et apte à se déplacer par rapport à un coulisseau (70,270) fixé à une extrémité de la barre, de manière à obtenir un mouvement de va-et-vient longitudinal du carter par rapport à la barre et éviter tout déplacement angulaire;
b) le second support (2) est déplaçable longitudinalement par rapport au premier support au moyen dudit organe de déplacement (6,201); et
c) l'élément d'amortissement (90,290) coopère avec des moyens de réglage (120,291) de la force d'amortissement disposés extérieurement en bout du fixateur.

2. Fixateur externe selon la revendication 1, caractérisé en ce que le coulisseau (70, 270) comporte une partie de section polygonale (77, 277), destinée à coulisser par rapport au carter (50, 250).

3. Fixateur externe selon la revendication 2, caractérisé en ce que ladite partie de section polygonale (77, 277) coulisse dans un roulement à aiguilles (60,260) solidaire du carter et apte à faciliter le mouvement de va-et-vient entre le carter et le coulisseau.

4. Fixateur externe selon la revendication 3, caractérisé en ce que le carter (50,250) comporte des dégagements inclinés (56,256) destinés à recevoir des lardons obliques (57,257) de pente correspondante, dont une face plane est apte à coopérer avec le roulement à aiguilles (60,260).

5. Fixateur externe selon la revendication 4, caractérisé en ce que le carter (50,250) comporte à une extrémité une collerette (51,251) munie d'un taraudage apte à recevoir une rondelle (58,258) de maintien des lardons et de la cage à aiguilles dans le carter.

6. Fixateur externe selon la revendication 1, caractérisé en ce que des moyens (4) de réglage de l'amplitude du va-et-vient sont prévus entre le carter (50,250) et l'extrémité de la barre (100,200), pour limiter le mouvement de va-et-vient longitudinal.

7. Fixateur externe selon la revendication 1, caractérisé en ce que les supports (2,3) d'orientation des fiches sont constitués par des mâchoires (23,33) disposées de part et d'autre de la barre (100,200) coopérant avec un organe arqué de déflection (22,32) apte à autoriser le pivotement angulaire d'étaux (21,31) de serrage des fiches osseuses (25,35).

8. Fixateur externe selon la revendication 7, caractérisé en ce qu'un renvoi angulaire (37) est inséré entre l'organe arqué de déflection (22,32) et l'étau (21,31).

9. Fixateur externe selon la revendication 7, caractérisé en ce qu'une pièce intermédiaire de raccord (38) est insérée entre les mâchoires (23,33) et le carter (50).

10. Fixateur externe selon la revendication 1, caractérisé en ce que l'élément d'amortissement (90,290) est constitué par un élément élastique comprimé entre deux portées, la première (126,276) étant au moins indirectement solidaire de la barre, et la seconde (94,294) solidaire du carter.

11. Fixateur externe selon la revendication 10, caractérisé en ce que l'élément d'amortissement est un ressort boudin (96,296).

12. Fixateur externe selon la revendication 10, caractérisé en ce que l'une des portées (126,294) est solidaire d'un embout (120,291) déplaçable par rapport à un organe (98,280) muni de repères (99,283) gradués, aptes à indiquer des valeurs de forces.

13. Fixateur externe selon la revendication 1, caractérisé en ce que la barre (100,200) est de section circulaire ou polygonale.

14. Fixateur externe selon la revendication 13, caractérisé en ce que la barre (100,200) comporte extérieurement des graduations (11) permettant de mesurer le mouvement effectué au moyen de l'organe de déplacement.

15. Fixateur externe selon la revendication 13, caractérisé en ce que la barre (100) est de forme générale quadrangulaire avec des pans coupés à 20° de manière à assurer le positionnement du support (2) d'orientation des fiches.

16. Fixateur externe selon la revendication 1, caractérisé en ce que la barre (200) est formée de tubes télescopiques intérieur (210) et extérieur (220).

17. Fixateur externe selon la revendication 16, caractérisé en ce que l'organe de déplacement apte à assurer le déplacement de l'un des supports est constitué par une tige filetée (201) solidaire des tubes télescopiques intérieur (210) et extérieur (220).

18. Fixateur externe selon la revendication 1, caractérisé en ce que l'organe de déplacement apte à assurer le déplacement de l'un des supports est constitué par un organe de déplacement (6) extérieur à la barre (100).

19. Fixateur externe selon la revendication 18, caractérisé en ce que l'organe (6) comporte des moyens de fixation (601 à 603) à la barre (100) et des moyens (604 à 609) de déplacement longitudinal du dit support.

20. Fixateur externe selon la revendication 1, caractérisé en ce que le carter (50,250) comporte une paroi extérieure (52,252) au moins indirectement solidaire d'une tige centrale (80,279) apte à se déplacer dans l'ouverture centrale (78,278) du coulisseau (70,270) pour assurer une stimulation de biocompression passive.

## Patentansprüche

1. Externe Fixiervorrichtung für die Korrektur und die Reduktion von Knochenfragmenten, mit einer starren Stange (100, 200), die im wesentlichen parallel zu dem Knochen angeordnet ist, wenigstens zwei Trägern (2, 3) zum Ausrichten von Stiften (25, 35), die in die Knochenfragmente eingefügt werden, wobei der Abstand zwischen den Trägern mittels eines Verlagerungselementes (6, 201) einstellbar ist, das jegliche Drehung zwischen den Trägern verhindert, wobei die Stange ein Dämpfungselement (90, 290) für die Verlagerung in Längsrichtung aufweist, dadurch gekennzeichnet, daß:
a) der erste Träger (3) einstückig mit einem Gehäuse (50, 250) ist, das unter der starren Stange (100, 200) eingerichtet ist und dazu ausgelegt ist, sich in bezug auf einen Schlitten (70, 270) zu verlagern, der an einem Ende der Stange angebracht ist, um so einen Hin- und Herbewegung in Längsrichtung des Gehäuses in bezug auf die Stange zu erreichen und jegliche winkelmäßige Versetzung auszuschalten;
b) der zweite Träger (2) in Längsrichtung in bezug auf den ersten Träger mittels des Verlagerungselementes (6, 201) verlagerbar ist; und
c) das Dämpfungselement (90, 290) mit einer Steuerungseinrichtung (120, 291) für die Dämpfungskraft zusammenwirkt, welche außerhalb unter der Fixiervorrichtung angeordnet ist.

2. Externe Fixiervorrichtung nach Anspruch 1, dadurch gekennzeichnet, daß der Schlitten (70, 270) einen Teil mit polygonalem Querschnitt (77, 277) aufweist, dazu bestimmt, in bezug auf das Gehäuse (50, 250) zu gleiten.

3. Externe Fixiervorrichtung nach Anspruch 2, dadurch gekennzeichnet, daß der Teil mit polygonalem Querschnitt (77, 277) in einem Nadellager (60, 260) gleitet, das einstückig mit dem Gehäuse gebildet ist und dazu ausgelegt ist, die Hin- und Herbewegung des Gehäuses und des Schlittens zu erleichtern.

4. Externe Fixiervorrichtung nach Anspruch 3, dadurch gekennzeichnet, daß das Gehäuse (50, 250) geneigte freie Bereiche (56, 256) aufweist, die dazu bestimmt sind, schräge Sperrleisten (57, 257) mit entsprechender Steigung aufzunehmen, deren eine Ebene dazu ausgelegt ist, mit dem Nadellager (60, 260) zusammenzuwirken.

5. Externe Fixiervorrichtung nach Anspruch 4, dadurch gekennzeichnet, daß das Gehäuse (50, 250) an einem Ende einen Kragen (51, 251) aufweist, der mit einem Gewinde versehen ist, daß dazu ausgelegt ist, eine Scheibe (58, 258) zum Halten der Sperrleisten und des Nadelkäfigs in dem Gehäuse aufzunehmen.

6. Externe Fixiervorrichtung nach Anspruch 1, dadurch gekennzeichnet, daß die Einrichtung (4) zum Steuern der Amplitude der Hin- und Herbewegung zwischen dem Gehäuse (50, 250) und dem Ende der Stange (100, 200) vorgesehen sind, um die Hin- und Herbewegung in Längsrichtung zu begrenzen.

7. Externe Fixiervorrichtung nach Anspruch 1, dadurch gekennzeichnet, daß die Träger (2, 3) zum Ausrichten der Stifte durch Klemmen (23, 33) gebildet sind, die beidseitig der Stange (100, 200) angeordnet sind und mit einem gekrümmten Ablenkelement (22, 32) zusammenwirken, daß dazu ausgelegt ist, die Winkel-Schwenkbewegung von Spannstücken (21, 31) zum Verriegeln der Knochenstifte (25, 35) zu erlauben.

8. Externe Fixiervorrichtung nach Anspruch 7, dadurch gekennzeichnet, daß ein Winkelgetriebe (37) zwischen dem gekrümmten Ablenkelement (22, 32) und dem Spannstock (21, 31) eingefügt ist.

9. Externe Fixiervorrichtung nach Anspruch 7, dadurch gekennzeichnet, daß ein Zwischenstück (38) zwischen die Klemmen (23, 33) und das Gehäuse (50) eingeschaltet ist.

10. Externe Fixiervorrichtung nach Anspruch 1, dadurch gekennzeichnet, daß das Dämpfungselement (90, 290) aus einem zusammengedrückten elastischen Element zwischen zwei Auflageflächen gebildet ist, wobei die erste (126, 276) wenigstens indirekt einstückig mit der Stange und die zweite (94, 294) einstückig mit dem Gehäuse ausgebildet ist.

11. Externe Fixiervorrichtung nach Anspruch 10, dadurch gekennzeichnet, daß das Dämpfungselement eine Schraubenfeder (96, 296) ist.

12. Externe Fixiervorrichtung nach Anspruch 10, dadurch gekennzeichnet, daß die eine der Auflageflächen (126, 294) einstückig mit einem Ansatzstück (120, 291) ist, das in bezug auf ein Element (98, 280) verlagerbar ist, das mit abgestuften Markierungen (99, 283) versehen ist, die dazu geeignet sind, die Kraftwerte anzugeben.

13. Externe Fixiervorrichtung nach Anspruch 1, dadurch gekennzeichnet, daß die Stange (100, 200) einen kreisförmigen oder polygonalen Querschnitt hat.

14. Externe Fixiervorrichtung nach Anspruch 13, dadurch gekennzeichnet, daß die Stange (100, 200) Außenabstufungen (11) aufweist, die es erlauben, die Bewegung zu messen, die zwischen den Verlagerungsorganen bewirkt wird.

15. Äußere Fixiervorrichtung nach Anspruch 13, dadurch gekennzeichnet, daß die Stange (100) im allgemeinen eine viereckige Form hat, mit auf 20° geschnittenen Seitenflächen, so daß die Positionierung des Trägers (2) zum Ausrichten der Stifte sichergestellt ist.

16. Externe Fixiervorrichtung nach Anspruch 1, dadurch gekennzeichnet, daß die Stange (20) aus teleskopischen Innen- (210) und Außen- (220) Rohren gebildet ist.

17. Externe Fixiervorrichtung nach Anspruch 16, dadurch gekennzeichnet, daß das Verlagerungselement, das dazu ausgelegt ist, die Verlagerung eines des Träger sicherzustellen, aus einer Gewindestange (201) gebildet ist, die einstückig mit den teleskopischen Innen- (210) und Außen- (220) Rohren ausgebildet ist.

18. Externe Fixiervorrichtung nach Anspruch 1, dadurch gekennzeichnet, daß das Verlagerungselement, das dazu ausgelegt ist, die Verlagerung eines der Träger sicherzustellen, aus einem Verlagerungselement (6) außerhalb der Stange (100) gebildet ist.

19. Externe Fixiervorrichtung nach Anspruch 18, dadurch gekennzeichnet, daß das Element (6) eine Fixiereinrichtung (601 -603) an die Stange (100) und Mittel (604 - 609) zum Verlagern des Trägers in Längsrichtung aufweist.

20. Externe Fixiervorrichtung nach Anspruch 1, dadurch gekennzeichnet, daß das Gehäuse (50, 250) eine Außenwand (52, 252) aufweist, die wenigstens indirekt einstückig mit einer Mittelstange (80, 279) ausgebildet ist, die dazu geeignet ist, sich in einer Mittelöffnung (78, 278) des Schlittens (70, 270) zu verlagern, um eine passive Biokompressionsstimulation zu gewährleisten.

## Claims

1. External fixator for the correction and reduction of bone fragments, which comprises a rigid bar (100,200) disposed substantially parallel to the bone, at least two supports (2,3) for the orientation of the pins (25,35) inserted into the bone fragments, the distance between said supports being adjustable with the aid of a displacement member (6,201) preventing any rotation between the supports, while the bar is provided with a member (90,290) damping the longitudinal displacement, characterized by the fact that:
a) the first support (3) is fastened to a casing (50,250) which is disposed at an end of the rigid bar (100,200) and adapted to move relative to a sliding block (70,270) fixed to one end of the bar, in such a manner as to obtain a longitudinal reciprocating movement of the casing relative to the bar and to avoid any angular displacement;
b) the second support (2) is displaceable longitudinally relative to the first one with the aid of said displacement member (6,201), and
c) the damping member (90,290) cooperates with damping force adjustment means (120,291) disposed outside the end of the fixator.

2. External fixator according to Claim 1, characterized in that the sliding block (70,270) has a portion of polygonal section (77,277) intended to slide relative to the casing (50,250).

3. External fixator according to Claim 2, characterized in that said portion of polygonal section (77,277) slides in a needle bearing (60,260) fastened to the casing and adapted to facilitate the reciprocating movement between the casing and the sliding block.

4. External fixator according to Claim 3, characterized in that the casing (50,250) is provided with inclined cutouts (56,256) intended to receive oblique shims (57, 257) which have a corresponding slope and of which a plane face is adapted to cooperate with the needle bearing (60,260).

5. External fixator according to Claim 4, characterized in that the casing (50,250) is provided at one end with a collar (51,251) having an internal screwthread adapted to receive a washer (58,258) securing the shims and the needle cage in the casing.

6. External fixator according to Claim 1, characterized in that means (4) for adjusting the length of the reciprocating movement are provided between the casing (50,250) and the end of the bar (100,200) in order to limit the longitudinal reciprocating movement.

7. External fixator according to Claim 1, characterized in that the supports (2,3) orienting the pins consist of jaws (23,22) disposed one on each side of the bar (100, 200) and cooperating with a curved deflection member (22,32) adapted to allow the angular pivoting of clamps (21,31) gripping the bone pins (25,35).

8. External fixator according to Claim 7, characterized in that an angle drive (37) is inserted between the curved deflection member (22,32) and the clamp (21,31).

9. External fixator according to Claim 7, characterized in that an intermediate connection member (38) is inserted between the jaws (23,33) and the casing (50).

10. External fixator according to Claim 1, characterized in that the damping member (90,290) consists of an elastic member compressed between two bearing surfaces, of which one (126,276) is at least indirectly fastened to the bar and the other (94,294) to the casing.

11. External fixator according to Claim 10, characterized in that the damping member is a coil spring (96,296).

12. External fixator according to Claim 10, characterized in that one of the bearing surfaces (126,294) is connected to an end-piece (120,291) which is displaceable with respect to a part (98,280) provided with graduated reference markings (99,283) adapted to indicate values of forces.

13. External fixator according to Claim 1, characterized in that the bar (100,200) has a circular or a polygonal cross-section.

14. External fixator according to Claim 13, characterized in that the outer face of the bar (100,200) is provided with graduations (11) making it possible to measure the mouvement effected by means of the displacement member.

15. External fixator according to Claim 13, characterized in that the bar (100) has a generally quadrangular shape with edges cut off at 20° so as to effect the positioning of the pin orientation support (2).

16. External fixator according to Claim 1, characterized in that the bar (200) is composed of inner (210) and outer (220) telescopic tubes.

17. External fixator according to Claim 16, characterized in that the displacement member adapted to effect the displacement of one of the supports consists of a threaded shaft (201) cooperating with inner (210) and outer (220) tubes.

18. External fixator according to Claim 1, characterized in that the displacement member adapted to effect the displacement of one of the supports consists of a displacement member (6) outside the bar (100).

19. External fixator according to Claim 18, characterized in that the member (6) is provided with means (601 to 603) for fastening it to the bar and with means (604 to 609) for the longitudinal displacement of said support.

20. External fixator according to Claim 1, characterized in that the casing (50,250) has an end wall (52,252) at least indirectly fixed to a central rod (80,279) adapted to slide in a central passage (78,278) in the sliding member (70,270), to ensure a passive biocompression stimulation.
